# EUROPEAN PATENT SPECIFICATION

(11) **EP 2 114 147 B1**
(45) Date of publication and mention of the grant of the patent: **16.05.2012**
(21) Application number: 08729655.4
(22) Date of filing: 12.02.2008
(51) Int. Cl.: A61K 31/135, A01N 33/02, A61K 31/519, A61K 31/485, A61P 15/10

(54) **REDUCING SIDE EFFECTS OF TRAMADOL**
VERRINGERUNG DER NEBENWIRKUNGEN VON TRAMADOL
RÉDUCTION DES EFFETS SECONDAIRES DU TRAMADOL

(30) Priority: 12.02.2007 US 889380 P
(43) Date of publication of application: 11.11.2009
(73) Proprietor: DMI Biosciences, Inc., Greenwood Village, CO 80111 (US)
(72) Inventor: BAR-OR, David, Englewood, CO 80110 (US); BILYARD, Kevin, Cheshire SK9 5PL (GB); WINKLER, James, V., Denver, CO 80203 (US)
(74) Representative: Thomas, Simon
(86) International application number: PCT/US2008/053722
(87) International publication number: WO 2008/100933

(56) References cited:
- EP-A2- 0 960 621
- WO-A1-2008/100926
- WO-A1-2008/131256
- WO-A2-03/072043
- WO-A2-2007/070779
- US-A- 6 087 362
- US-A1- 2003 031 712
- US-A1- 2004 087 591
- US-A1- 2005 054 688
- US-A1- 2006 092 691
- US-B1- 6 403 597
- US-B2- 6 974 839
- WERNEKE ET AL.: 'Antidepressants and sexual dysfunction' ACTA PSYCHIATRICA SCANDINAVICA vol. 114, December 2006, pages 384 - 397, XP008115962

## Description

### FIELD OF THE INVENTION

The invention relates to pharmaceutical compositions for reducing the side effects of tramadol. Side effects related to sexual function, such as erectile dysfunction, are newly discovered side effects of tramadol. Accordingly, in one embodiment, the invention relates to a pharmaceutical compositions for reducing the incidence of one or more side effects related to sexual function in human males taking tramadol. In one embodiment, the composition comprises a tramadol material and a phosphodiesterase inhibitor. The invention further relates to kits. In one embodiment, the kit comprises a tramadol material and a phosphodiesterase inhibitor.

### BACKGROUND OF THE INVENTION

Tramadol is a centrally acting synthetic analgesic compound. Its mode of action is not completely understood. From animal tests, at least two complementary mechanisms appear applicable: (1) the binding of the parent compound (tramadol) and the O-demethylated M1 metabolite to µ-opioid receptors; and (2) a weak inhibition of reuptake of norepinephrine and serotonin. Opioid activity is due to both low affinity binding of the parent compound and higher affinity binding of the M1 metabolite to µ-opioid receptors. In animal models, M1 is up to 6 times more potent than tramadol in producing analgesia and 200 times more potent in µ-opioid binding. Tramadol has been shown to inhibit reuptake of norepinephrine and serotonin *in vitro,* as have some other opioid analgesics. These mechanisms may contribute independently to the overall analgesic profile of tramadol.

Apart from analgesia, the use of tramadol to treat frequent urination and urinary incontinence (see U.S. Patent No. 6,090,856), to treat coughs, bronchitis and the common cold (see U.S. Patents Nos. 3,652,589 and 3,830,934) and to treat premature ejaculation (U.S. Patent No. 6,974,839) have been described.

Tramadol has known side effects when used for analgesia, especially when used for long periods of time to treat chronic pain. Reported side effects with an incidence of 5% or greater are dizziness, nausea, constipation, headache, somnolence, vomiting, pruritis, CNS stimulation (nervousness, anxiety, agitation, tremor, spasticity, euphoria, emotional lability and hallucinations), asthenia, sweating, dyspepsia, dry mouth and diarrhea. See Physicians' Desk Reference, entry for Ultram^{®} tramadol hydrochloride. Methods of reducing these side effects of tramadol have been described. See U.S. Patents Nos. 6,056,968, 6,221,394, 6,297,286, 6,696,066 and 6,765,010, U.S. Patent Appl. Pubs. Nos. 2001/0006967 and 2006/0052389, and PCT applications WO 00/32558 and WO 00/67739.

### SUMMARY OF THE INVENTION

It has recently been found that human males taking (±)-cis-tramadol hydrochloride experienced side effects related to sexual function (see Examples 1-2 below). These side effects were erectile dysfunction (8% - 14%), anorgasmia (3% - 7%), penile hypoesthesia (4% - 5%), decreased libido (< 1% - 2%) and decreased orgasmic sensation (0 - < 1 %). These males were taking a single dose of *(*±*)*-cis-tramadol in the lower therapeutic range for analgesia. Since the number of males experiencing side effects related to sexual function increased with the dose of *(*±*)*-cis-tramadol, the percentage of these side effects may be even higher in males taking higher doses of tramadol or taking tramadol chronically. These side effects of tramadol were not previously known.

Accordingly, the invention provides a pharmaceutical composition comprising a pharmaceutically-acceptable carrier and active ingredients, wherein the active ingredients consist of a tramadol material and a phosphodiesterase (PDE) inhibitor selected from one or more of a PDE3, PDE4 and a PDE5 inhibitor.

The invention also provides a pharmaceutical composition comprising a pharmaceutically-acceptable carrier, a tramadol material, a phosphodiesterase (PDE) inhibitor selected from one or more of a PDE3, PDE4 and a PDE5 inhibitor, and an opioid antagonist.

The invention also provides a kit wherein:
(a) the kit comprises a container holding active ingredients consisting of (i) a tramadol material and (ii) a phosphodiesterase (PDE) inhibitor selected from one or more of a PDE3, PDE4 and PDE5 inhibitor; or
(b) the kit consists of a first container holding a tramadol material and a second container holding a phosphodiesterase (PDE) inhibitor selected from one or more of a PDE3, PDE4 and PDE5 inhibitor.

The invention also provides a kit comprising one or more containers, wherein each container holds a tramadol material, a phosphodiesterase (PDE) inhibitor, and an opioid antagonist

The invention also provides a pharmaceutical composition comprising a pharmaceutically-acceptable carrier and active ingredients, wherein the active ingredients consist of (i) one or more of a PDE3, PDE4 and PDE5 inhibitor and (ii) a tramadol material, for use in reducing the incidence of a side effect related to sexual function in a human male taking a tramadol material wherein the side effect is caused by the administration of a tramadol material.

The invention also provides a pharmaceutical composition comprising a pharmaceutically-acceptable carrier and active ingredients, wherein the active ingredients consist of (i) one or more of a PDE3, PDE4 and PDE5 inhibitor and (ii) a tramadol material, for use in the treatment of a disease or condition in a human male for which a tramadol material is an effective treatment, wherein the disease or condition is selected from pain, frequent urination, urinary incontinence, cough, bronchitis, common cold or premature ejaculation.

The invention also provides a pharmaceutical composition comprising a pharmaceutically-acceptable carrier, one or more of a PDE3, PDE4 and PDE5 inhibitor, a tramadol material, and an opioid antagonist for use in the treatment or a disease or condition in a human male for which a tramadol material is an elective treatment, wherein the disease or condition is selected from pain, frequent urination, urinary incontinence, cough, bronchitis, common cold or premature ejaculation.

The invention also provides a pharmaceutical composition comprising a pharmaceutically-acceptable carrier, a phosphodiesterase (PDE) inhibitor selected from one or more of a PDE3, PDE4 and PDE5 inhibitor, an opioid antagonist, and a tramadol material for use in delaying ejaculation in a human male.

### BRIEF DESCRIPTION OF THE DRAWINGS

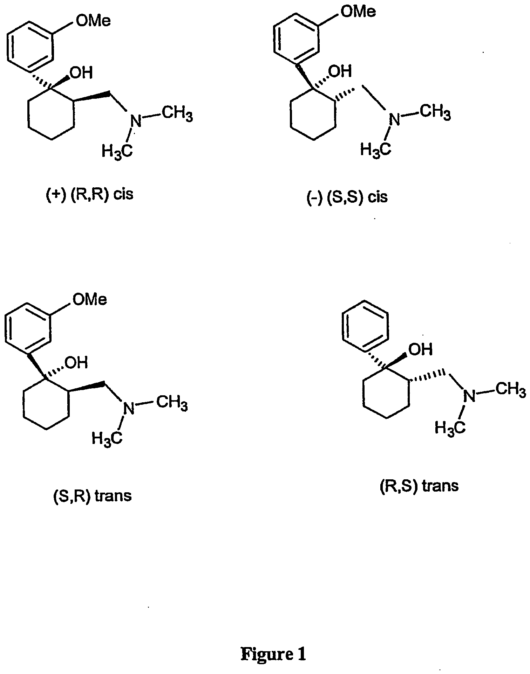

Figure 1 shows stereoisomers of tramadol.

### DETAILED DESCRIPTION OF THE PRESENTLY-PREFERRED EMBODIMENTS OF THE INVENTION

As used herein, tramadol "side effects related to sexual function" include erectile dysfunction, anorgasmia, penile hypoesthesia, decreased libido and decreased orgasmic sensation.

As used herein, tramadol "side effects not related to sexual function" include dizziness, nausea, constipation, headache, somnolence, fatigue, insomnia, vomiting, pallor, pruritis, nervousness, anxiety, hyperactivity, restlessness, agitation, tremor, spasticity, euphoria, emotional lability, disturbance in attention, depressed mood, euphoric mood, tremor, hallucinations, anosmia, nasal congestion, asthenia, sweating, dyspepsia, dry mouth, itching, flatulence and diarrhea. For others, see *Physicians'Desk Reference,* entry for Ultram® tramadol hydrochloride.

As used herein, "reduce the incidence of a side effect" means that the side effect will be prevented or that the number of incidences of the side effect will be reduced.

As used herein, the term "erectile dysfunction" means the consistent or recurrent inability to achieve and/or maintain a penile erection sufficient to permit satisfactory sexual intercourse or activity. "Erectile dysfunction" is also used herein to mean the partial, temporary or episodic absence of a penile erection.

The erectile function (EF) domain of the International Index of Erectile Function (IIEF) has been developed and validated as a patient-based questionnaire that is now widely use for the diagnosis of erectile dysfunction. The EF domain of the IIEF has demonstrated test reliability and validity with a high degree of sensitivity and specificity. In particular, men scoring 25 or less are classified as having ED and those scoring above 25 are classified as not having ED (sensitivity = 0.97; specificity = 0.88). Further, responses to IIEF erectile function questions can classify erectile dysfunction into five diagnostic categories: no erectile dysfunction (score 26-30); 'mild' erectile dysfunction (score 22-25); 'mild-to-moderate' erectile dysfunction (score 17-21); 'moderate' erectile dysfunction (score 11-16); and 'severe' erectile dysfunction (score 6-10). See Rosen, et al., Int. J. Impot. Res., 14(4):226-244 (August 2002) and Rosen, et al., Urology, 49:822-830 (1997) (includes a copy of the IIEF questionnaire and identification of the EF questions). Another subset of the IIEF called the Sexual Health Inventory for Men (SHIM) was also developed and validated as a diagnostic tool that is now widely use for the diagnosis of erectile dysfunction. Men scoring 21 or less are classified as having ED and those scoring above 21 are classified as not having ED (sensitivity = 0.98; specificity = 0.88). Further, responses to SHIM questions can classify erectile dysfunction into five diagnostic categories: no erectile dysfunction (score 22-25); 'mild' erectile dysfunction (score 17-21); 'mild-to-moderate' erectile dysfunction (score 12-16); 'moderate' erectile dysfunction (score 8-11); and 'severe' erectile dysfunction (score 5-7). See Rosen, et al., Int. J. Impot. Res., 14(4):226-244 (August 2002) (identifies the SHIM questions) and Rosen, et al., Urology, 49:822-830 (1997) (includes a copy of the IIEF questionnaire). Accordingly, erectile dysfunction can be diagnosed using the EF score and/or the SHIM score, and the severity of erectile dysfunction can also be assessed using these scores.

As used herein, "reduce the incidence of erectile dysfunction" means that erectile dysfunction will be prevented or that the number of incidences of erectile dysfunction will be reduced. As used herein, "reduce the severity of erectile dysfunction" means that the severity of erectile dysfunction as measured by the EF and/or SHIM score is reduced (*i.e.,* the EF or SHIM score increases).

As used herein, the term "premature ejaculation" means a sexual dysfunction wherein a male is unable to control the ejaculatory process to a degree sufficient to satisfy a partner and/or himself. Premature ejaculation refers to persistent or recurring ejaculation with minimal stimulation and/or that occurs sooner than desired, before or shortly after penetration during sexual intercourse, causing distress to one or both partners. See Montague, et al. J. Urol., 172:290-294 (2004); Diagnostic and Statistical Manual of Mental Disorders, 4th ed., American Psychiatric Association, Washington, D.C. (2000). The term includes "congenital," "lifelong," "primary" and "acquired" premature ejaculation.

Although a variety of specific criteria have been proposed for diagnosing premature ejaculation, no one criterion or group of criteria is yet universally accepted. Specific proposed criteria include: (i) ejaculation prior to penetration or within ten to twenty strokes after intromission; (ii) ejaculation in less than 1-2 minutes; and (iii) ejaculation 50% of the time more rapidly than the female is able to have an orgasm if she has no orgasmic dysfunction. See, *e.g*., U.S. Patent No. 6,037,360 and 5,151,448; Male Infertility and Sexual Dysfunction, page 356 (Springer-Verlag 1997); Diagnostic and Statistical Manual of Mental Disorders (American Psychiatric Association 1994). More recently, an intravaginal ejaculatory latency time ('IELT' or 'IVELT') measured by stopwatch of less than 2 minutes combined with evidence of distress or interpersonal difficulty has been used for the diagnosis of premature ejaculation in clinical studies. See Pryor, et al., Lancet, 368(9539):929-937 (September 9, 2006). One report suggests that men with an IELT of less than 1 minute have "definite" premature ejaculation and that men with an IELT between 1 and 1.5 minutes have "probable" premature ejaculation, whereas the severity of the premature ejaculation (such as "non-symptomatic," "mild," "moderate" and "severe") should be defined in terms of associated psychological problems. Waldinger, et al., J. Sex. Med., 2(4):498-507 (2005). Various self-reported outcome questionnaires, also referred to as patient-reported outcomes, for diagnosing premature ejaculation have been developed. See Althof, et al., Urol. Clin. North Am., 34(4):581-589 (November 2007). The Premature Ejaculation Diagnostic Tool (PEDT) is one such questionnaire. It has recently been validated and indicates that scores of 9 and 10 are "probable" premature ejaculation and scores equal to or greater than 11 are diagnostic of premature ejaculation. See Symonds et al., Eur. Urol., 52:565-573 (2007) and Symonds et al., Int. J. Impot. Res., 19:521-5 (2007) (includes a copy of the questionnaire). This questionnaire evaluates lack of control, frequency of premature ejaculation, minimal sexual stimulation, distress and interpersonal difficulties. Presently, the inventors consider that the best criteria for diagnosing premature ejaculation are a short IELT plus a PEDT score of 9 or greater. The exact definition of a short IELT is expected to vary depending on geographic area and/or cultural differences, and can be determined empirically. For the United States, the inventors presently consider that the best definition of a short IELT is an IELT of less than 2 minutes in greater than 50% of coital attempts as measured using a stopwatch.

As used herein, "delay ejaculation" means that a male receiving treatment is able to control the ejaculatory process so as to prevent ejaculation for a time which is longer than that normally experienced by the male when not receiving treatment. It is expected that the male will be able to control the ejaculatory process to a degree sufficient to better or completely satisfy his partner. "Delay ejaculation" does not mean to totally prevent ejaculation.

The term "tramadol material" is used herein to refer to 2-[(dimethylamino)methyl]-1-(3-methoxyphenyl)-cyclohexanol ("tramadol") and all pharmaceutically-acceptable forms and derivatives of tramadol. In particular, the term includes the N-oxide derivative ("tramadol N-oxide") and the O-desmethyl derivative ("O-desmethyl tramadol"). The term also includes the solvates, polymorphs, and pharmaceutically-acceptable acid addition salts of tramadol and its derivatives. The term further includes all of the stereoisomers of any of the foregoing, including individual stereoisomers (including individual enantiomers) and mixtures of stereoisomers (including the racemates).

The stereoisomers of tramadol are shown in Figure 1. There appears to be some discrepancy in the literature regarding the nomenclature of the individual stereoisomers of tramadol. For the purposes of the present application, the designations of "cis" and "trans" stereoisomers of tramadol are made in reference to the relative positions of the dimethylamino and the hydroxy substituents on the cyclohexane ring within the tramadol molecule. As shown in Figure 1, the R,R and S,S enantiomers will be referred to herein as the "cis" isomers while the R,S and S,R isomers will be referred to herein as the "trans" isomers. As also shown in Figure 1, the R,R isomer of tramadol will be referred to herein as the "+" cis isomer and the S,S isomer will be referred to as the "-" cis isomer. It is presently understood that R,S and S,R isomers are not optically active.

Methods of making tramadol, tramadol N-oxide, and O-desmethyl tramadol are well known. See, *e.g*., U.S. Patents Nos. 3,652,589, 3,830,934, 5,223,541, 5,336,691, 5,723,668, 5,728,885, and 5,874,620. Tramadol is also commercially available from several sources, including Gruenenthal GmbH, Aschen, Germany.

The pharmaceutically-acceptable acid addition salts are prepared by conventional methods well known in the art using pharmaceutically-acceptable, substantially non-toxic, organic and inorganic acids. Such acids include hydrochloric acid, nitric acid, sulfuric acid, phosphoric acid, hydrobromic acid, acetic acid, propionic acid, maleic acid, malonic acid, succinic acid, citric acid, tartaric acid, malic acid, benzoic acid, salicylic acid, phthalic acid, nicotinic acid, etc. Preferred is hydrochloric acid.

Presently preferred is tramadol and the acid addition salts thereof, particularly the hydrochloride. More preferred is (±)*cis*-tramadol, the acid addition salts thereof (particularly the hydrochloride), the individual enantiomers (particularly the (-)enantiomer), and non-racemic tramadol comprising at least 60% of the (-)enantiomer.

Disease and conditions for which treatment with a tramadol material is indicated include pain, frequent urination, urinary incontinence, coughs, bronchitis, the common cold and premature ejaculation. To treat such a disease or condition, an effective amount of a tramadol material is administered. By an "effective amount" is meant a nontoxic, but sufficient, amount of a tramadol material to treat the disease or condition. "Treat" is used herein to mean to reduce (wholly or partially) the symptoms of a disease or condition, including curing the disease or condition, or to prevent the disease or condition. Effective dosage forms, modes and times of administration, and amounts of a tramadol material to treat a disease or condition are known or can be determined empirically. Effective amounts of a tramadol material for treating pain are well known. See, *e.g., Physicians' Desk Reference,* entry for Ultram® tramadol hydrochloride. Effective amounts of a tramadol material to treat frequent urination, urinary incontinence, coughs, bronchitis or the common cold have been described. See, *e.g*., U.S. Patents Nos. 6,090,856, 3,652,589 and 3,830,934. An effective amount of *(*±*)cis*-tramadol HCl to delay ejaculation or treat premature ejaculation is from about 1 mg to about 250 mg, preferably from about 10 mg to about 200 mg, more preferably from about 25 mg to about 150 mg, given orally from about 30 minutes to about 24 hours before sexual activity. However, it is understood by those skilled in the art that the dosage amount will vary with the particular form of tramadol employed, the route(s) of administration, the timing of the administration, the identity of any other drugs being administered, the disease or condition being treated, the severity of the disease or condition, the age, size and condition of the patient, and like factors known in the medical art. In general, a suitable dose will be that amount of the compound which is the lowest dose effective to delay ejaculation without toxicity. However, the dosage, route of administration, etc., will be determined by an attending physician within the scope of sound medical judgment.

Phosphodiesterases (PDEs) are a class of intracellular enzymes involved in the metabolism of the second messenger nucleotides cAMP and cGMP. The PDEs have now been classified into eleven major families, Types I-XI. The members of the families vary in their tissue, cellular and subcellular distribution, as well as their links to the cAMP and cGMP pathways. For example, PDE type III (PDE3), PDE type IV (PDE4) and PDE type V (PDE5) are found in the corpus cavernosum, with PDE5 being the most abundant.

A "phosphodiesterase inhibitor" is an agent that is capable of inhibiting or reducing, selectively or nonselectively, the activity of a PDE. Suitable PDE inhibitors for use in the present invention include those described in U.S. Patents Nos. 5,250,534, 5,859,006, 6,140,329, 6,362,178,6,403,597,6,469,012,6,821,975,6,943,166 and 6,943,171. Methods of making PDE inhibitors are known. See U.S. Patents Nos. 5,250,534, 5,859,006, 6,140,329, 6,362,178, 6,403,597, 6,469,012, 6,821,975, 6,943,166 and 6,943,171. The PDE inhibitor used in the present invention is preferably an inhibitor of PDE3, PDE4 and/or PDE5. More preferably, the PDE inhibitor is a selective inhibitor of PDE5. Even more preferably, the inhibitor is sildenafil, vardenafil and/or tadalafil, and pharmaceutically-acceptable forms (*e.g.*, salts, solvates, stereoisomers (individual isomers and mixtures of isomers), etc.) of them. Most preferably, the inhibitor is sildenafil citrate (*e.g*., Viagra® sildenafil citrate; Pfizer), vardenafil hydrochloride (*e.g.*, Levitra® vardenafil HCl; Schering-Plough) and/or tadalafil (*e.g.*, Cialis®; Lilly ICOS).

To reduce the incidence of a side effect related to sexual function in a human male taking a tramadol material, an effective amount of a phosphodiesterase inhibitor is administered to the male in addition to the tramadol material. The phosphodiesterase inhibitor must, of course, be administered an effective time prior to sexual activity. By an "effective time" is meant the range of time prior to sexual activity during which the phosphodiesterase inhibitor must be administered so that it will be effective to reduce the incidence of the side effect(s). Of particular concern is erectile dysfunction as a side effect of administration of a tramadol material, and the invention is especially concerned with reducing the incidence and/or severity of erectile dysfunction in males taking a tramadol material. The tramadol material and the phosphodiesterase inhibitor may be administered simultaneously or sequentially in any order. They may be administered separately, by the same or different modes of administration, or they may be administered in combination in a single dosage form by a single route of administration. By an "effective amount" is meant a nontoxic, but sufficient, amount of a phosphodiesterase inhibitor to reduce the incidence of a side effect related to sexual function in human males taking a tramadol material. An effective amount of Viagra® sildenafil citrate is from about 5 mg to about 500 mg, preferably from about 25 mg to about 100 mg, given orally from about 30 minutes to about 4 hours before sexual activity. An effective amount of Levitra® vardenafil hydrochloride is from about 1 mg to about 100 mg, preferably from about 5 mg to about 20 mg, given orally from about 30 minutes to about 2 hours before sexual activity. An effective amount of Cialis® tadalafil is from about 1 mg to about 100 mg, preferably from about 5 mg to about 20 mg, given orally from about 30 minutes to about 36 hours before sexual activity. However, it is understood by those skilled in the art that the dosage amount will vary with the particular form and amount of tramadol employed, the reason for the administration of the tramadol, the particular phosphodiesterase inhibitor employed, the route(s) of administration, the timing of the administration, the identity of any other drugs being administered, the severity of the side effect(s), the age, size and condition of the patient, and like factors known in the medical art. In general, a suitable dose will be that amount of the compound which is the lowest dose effective to reduce the incidence of the side effect(s) without toxicity. However, the dosage, route of administration, etc., will be determined by an attending physician within the scope of sound medical judgment. Effective dosage forms, modes and times of administration, and dosage amounts can be determined empirically.

Suitable second side-effect-reducing agents for reducing the incidence of a side effect of tramadol which is not related to sexual function are known. See, *e.g*., U.S. Patents Nos. 6,056,968, 6,221,394, 6,297,286, 6,696,066 and 6,765,010, U.S. Patent Appl. Pubs. Nos. 2001/0006967 and 2006/0052389, and PCT applications WO 00/32558 and WO 00/67739, the complete disclosure of each of which is incorporated herein by reference. The invention also includes the use of additional such agents which are developed hereafter.

As used herein, a "second side-effect-reducing agent" is an opioid antagonist that is able to reduce the incidence of one or more of the side effects of a tramadol material not related to sexual function.

The second side-effect-reducing agent is an opioid antagonist, as described in U.S. Patent No. 6,765,010, U.S. Patent Appl. Pubs. Nos. 2001/0006967 and 2006/0052389, and PCT application WO 00/67739. Suitable opioid antagonists include nalmefene, naltrexone, naloxone, etorphine and dihydroetorphine. Preferred for use herein is naltrexone. In particular, these references teach that a small amount of an opioid antagonist (*e.g*., 10 ng to 1 mg) enhances tramadol's analgesia while reducing tramadol's side effects. These references also teach that using larger amounts of the opioid antagonist will reduce the effectiveness of tramadol. However, quite surprisingly, it has been found by the present inventors that use of amounts of naltrexone from 2.5 mg to 10 mg did not reduce the effectiveness of tramadol in delaying ejaculation (see Examples 1-2).

U.S. Patents Nos. 6,056,968,6,221,394 and 6,297,286 and PCT application WO 00/32558 teach that the (-)enantiomer of a tramadol material reduces the side effects associated with (±)tramadol, including nausea, vomiting, constipation, dizziness, blurred vision, drowsiness, somnolence, sedation, hallucinations, respiratory depression and euphoria. In particular, the (- )enantiomer is antiemetic and reduces nausea and vomiting. Thus, to reduce the incidence of a side effect of tramadol other than erectile dysfunction, (-)tramadol can be administered in addition to a tramadol material. Alternatively, a non-racemic tramadol material comprising an increased amount, preferably at least 60% and up to 100%, of the (-)enantiomer of a tramadol material can be used as the tramadol material.

To reduce the incidence of a side effect of a tramadol material related to sexual function and the incidence of a side effect of a tramadol material not related to sexual function in a human male taking a tramadol material, an effective amount of a phosphodiesterase inhibitor and an effective amount of an opioid antagonist are administered to the male in addition A to the tramadol material. The three drugs may be administered simultaneously or sequentially in any order. They may be administered separately, by the same or different modes of administration, or they may be administered in combination in a single dosage form by a single route of administration. It is understood by those skilled in the art that the dosage amount will vary with the particular form and amount of tramadol employed, the reason for the administration of the tramadol, the particular second side-effect-reducing agent employed, the particular phosphodiesterase inhibitor employed, the route(s) of administration, the timing of the administration, the identity of any other drugs being administered, the severity of the side effects, the age, size and condition of the patient, and like factors known in the medical art. In general, a suitable dose will be that amount of the compound which is the lowest dose effective to reduce the incidence of a side effect related to sexual function and the incidence of a side effect not related to sexual function without toxicity. However, the dosage, route of administration, etc., will be determined by an attending physician within the scope of sound medical judgment. Effective dosage forms, modes and times of administration, and dosage amounts can be determined empirically.

The tramadol material, the phosphodiesterase inhibitor and the opioid antagonist may be administered by any suitable route of administration, including orally, nasally, rectally, parenterally (*e.g*., intravenously, subcutaneously, or intramuscularly), topically (*i.e*., delivery to the skin or mucosa), transdermally (*i.e.*, delivery by passage of a drug through the skin into the bloodstream), transmucosally (*i.e.*, delivery by passage of a drug through the mucosal tissue into the bloodstream), intracavernosally (*i.e*., injection into one or both corpora of the corpora cavernosal tissues of the penis), and intarurethrally (*i.e*., delivery into the urethra). Highly preferred is oral administration.

While it is possible for the tramadol material, the phosphodiesterase inhibitor and the opioid antagonist to be administered alone, it is preferable to administer them (individually or in various combinations) as a pharmaceutical formulation (composition). The pharmaceutical compositions may comprise a tramadol material and a phosphodiesterase inhibitor, and may also comprise an opioid antagonist, as the active ingredients in admixture with one or more pharmaceutically-acceptable carriers and, optionally, with one or more other compounds, or other materials. Each carrier must be "acceptable" in the sense of being compatible with the other ingredients of the formulation and not injurious to the male who will take the composition. Pharmaceutically-acceptable carriers are well known in the art. Regardless of the route of administration selected, the active ingredients are formulated into pharmaceutically-acceptable dosage forms by conventional methods known to those of skill in the art. See, *e.g., Remington's Pharmaceutical Sciences*

Pharmaceutical compositions containing a tramadol material and methods of making the pharmaceutical compositions have been described. See, *e.g*., U.S. Patents Nos. 3,652,589, 3,830,934, 5,223,541, 5,591,452, 5,601,842, 5,728,885, 6,017,963, 6,090,856, and 6,156,342, the complete disclosures of which are incorporated herein by reference. Moreover, pharmaceutical compositions containing tramadol and pharmaceutically-acceptable salts thereof are manufactured and sold worldwide. In the United States, (±) *cis*-2-[(dimethylamino)methyl]-1-(3-methoxyphenyl)-cyclohexanol hydrochloride for oral administration is available from Ortho-McNeil Pharmaceutical, Inc., Raritan, New Jersey 08869, as ULTRAM tablets. Each ULTRAM tablet contains 50 mg (±) *cis*-2-[(dimethylamino)methyl]-1-(3-methoxyphenyl)-cyclohexanol hydrochloride and a number of inactive ingredients (corn starch, hydroxypropyl methylcellulose, lactose, magnesium stearate, microcrystalline cellulose, polyethylene glycol, polysorbate 80, sodium starch glycolate, titanium dioxide and wax). It is understood the commercial preparation of tramadol marketed under the brand name ULTRAM^{®} consists of a mixture of the R,R and S,S isomers of tramadol hydrochloride.

Pharmaceutical compositions containing a phosphodiesterase inhibitor and methods of making the pharmaceutical compositions have also been described. See, *e.g*., U.S. Patents Nos. 5,250,534, 5,859,006, 6,140,329, 6,362,178, 6,403,597, 6,469,012, 6,821,975, 6,943,166 and 6,943,171, the complete disclosures of which are incorporated herein by reference. Suitable phosphosdiesterase inhibitors are also available commercially from, *e.g*, Pfizer (Viagra® sildenafil citrate), Schering-Plough (Levitra® vardenafil HCl) and Lilly ICOS (Cialis® tadalafil).

Formulations of the invention suitable for oral administration may be in the form of capsules, cachets, pills, tablets, powders, granules or as a solution or a suspension in an aqueous or non-aqueous liquid, or an oil-in-water or water-in-oil liquid emulsions, or as an elixir or syrup, or as pastilles (using an inert base, such as gelatin and glycerin, or sucrose and acacia), and the like, each containing a predetermined amount of the active ingredients. Preferred oral administration forms are tablets and capsules.

In solid dosage forms of the invention for oral administration (capsules, tablets, pills, dragees, powders, granules and the like), the active ingredients are mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monosterate; (8) absorbents, such as kaolin and bentonite clay; (9) lubricants, such as talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may be employed as fillers in soft and hard-filled gelatin capsules using such excipients as lactose or milk sugars, as well as high molecular weight polyethylene glycols and the like.

A tablet may be made by compression or molding optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or control led release of the active ingredients therein using, for example, hydroxypropylmethyl cellulose in varying proportions to provide the desired release profile, other polymer matrices, liposomes and/or microspheres. They may be sterilized by, for example, filtration through a bacteria-retaining filter. These compositions may also optionally contain opacifying agents and may be of a composition that they release the active ingredients only, or preferentially, in a certain portion of the gastrointestinal tract, optionally, in a delayed manner. These compositions may also be of a composition so that they release the active ingredients only, or preferentially, in a certain sequence (*e.g*., one before the other, one immediately and the other over time, both over time but with different release profiles, etc.). Examples of embedding compositions which can be used include polymeric substances and waxes. The active ingredients can also be in microencapsulated form.

Liquid dosage forms for oral administration of the compounds of the invention include pharmaceutically-acceptable emulsions, microemulsions, solutions, suspensions, syrups and elixirs. In addition to the active ingredients, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming, thickening, and preservative agents.

Suspensions, in addition to the active ingredients, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal administration may be presented as a suppository, which may be prepared by mixing the active ingredients with one or more suitable nonirritating excipients or carriers comprising, for example, cocoa butter, polyethylene glycol, a suppository wax or salicylate, and which is solid at room temperature, but liquid at body temperature and, therefore, will melt in the rectum and release the active ingredients.

Dosage forms for the topical, transdermal or transmucosal administration of the active ingredients include powders, sprays, ointments, pastes, creams, lotions, gels, solutions, patches, drops and inhalants. The active ingredients may be mixed under sterile conditions with a pharmaceutically-acceptable carrier, and with any buffers, or propellants which may be required.

The ointments, pastes, creams and gels may contain, in addition to the active ingredients, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the active ingredients, excipients such as lactose, talc, silicic acid, aluminum hydroxide, calcium silicates and polyamide powder or mixtures of these substances. Sprays can additionally contain customary propellants such as chlorofluorohydrocarbons and volatile unsubstituted hydrocarbons, such as butane and propane.

The active ingredients may also be delivered through the skin using conventional transdermal drug delivery systems, *i.e.,* transdermal patches, wherein the active ingredients are typically contained within a laminated structure that serves as a drug delivery device to be affixed to the skin. In such a structure, the active ingredients are typically contained in a layer, or "reservoir," underlying an upper backing layer. The laminated device may contain a single reservoir, or it may contain multiple reservoirs. In one embodiment, the reservoir comprises a polymeric matrix of a pharmaceutically acceptable contact adhesive material that serves to affix the system to the skin during drug delivery. Examples of suitable skin contact adhesive materials include, but are not limited to, polyethylenes, polysiloxanes, polyisobutylenes, polyacrylates, polyurethanes, and the like. Alternatively, the drug-containing reservoir and skin contact adhesive are present as separate and distinct layers, with the adhesive underlying the reservoir which, in this case, may be either a polymeric matrix as described above, or it may be a liquid or hydrogel reservoir, or may take some other form.

The backing layer in these laminates, which serves as the upper surface of the device, functions as the primary structural element of the laminated structure and provides the device with much of its flexibility. The material selected for the backing material should be selected so that it is substantially impermeable to the active ingredient and any other materials that are present. The backing layer may be either occlusive or nonocclusive, depending on whether it is desired that the skin become hydrated during drug delivery. The backing is preferably made of a sheet or film of a preferably flexible elastomeric material. Examples of polymers that are suitable for the backing layer include polyethylene, polypropylene, polyesters, and the like.

During storage and prior to use, the laminated structure includes a release liner. Immediately prior to use, this layer is removed from the device to expose the basal surface thereof, either the drug reservoir or a separate contact adhesive layer, so that the system may be affixed to the skin. The release liner should be made from a drug/vehicle impermeable material.

Transdermal drug delivery devices may be fabricated using conventional techniques, known in the art, for example by casting a fluid admixture of adhesive, drug and vehicle onto the backing layer, followed by lamination of the release liner. Similarly, the adhesive mixture may be cast onto the release liner, followed by lamination of the backing layer. Alternatively, the drug reservoir may be prepared in the absence of drug or excipient, and then loaded by "soaking" in a drug/vehicle mixture.

The laminated transdermal drug delivery systems may in addition contain a skin permeation enhancer. That is, because the inherent permeability of the skin to some drugs may be too low to allow therapeutic levels of the drug to pass through a reasonably sized area of unbroken skin, it is necessary to coadminister a skin permeation enhancer with such drugs. Suitable enhancers are well known in the art.

The pharmaceutical compositions of the invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, propellants such as fluorocarbons or nitrogen, and/or other conventional solubilizing or dispersing agents.

Preferred formulations for topical drug delivery are ointments and creams. Ointments are semisolid preparations which are typically based on petrolatum or other petroleum derivatives. Creams containing the selected active agents, are, as known in the art, viscous liquid or semisolid emulsions, either oil-in-water or water-in-oil. Cream bases are water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant. The specific ointment or cream base to be used, as will be appreciated by those skilled in the art, is one that will provide for optimum drug delivery. As with other carriers or vehicles, an ointment base should be inert, stable, nonirritating and nonsensitizing.

Formulations for buccal administration include tablets, lozenges, gels and the like. Alternatively, buccal administration can be effected using a transmucosal delivery system as known to those skilled in the art.

Pharmaceutical compositions suitable for parenteral administrations comprise the active ingredients in combination with one or more pharmaceutically-acceptable sterile isotonic aqueous or non-aqueous solutions, dispersions, suspensions or emulsions, or sterile powders or other solid forms which may be reconstituted into sterile injectable solutions or dispersions just prior to use, which may contain antioxidants, buffers, solutes which render the formulation isotonic with the blood of the intended recipient or suspending or thickening agents.

Examples of suitable aqueous and nonaqueous carriers which may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use ofsurfactants.

These compositions may also contain adjuvants such as wetting agents, emulsifying agents and dispersing agents. It may also be desirable to include isotonic agents, such as sugars, sodium chloride, and the like in the compositions. In addition, prolonged absorption of injectable pharmaceutical forms may be brought about by the inclusion of agents which delay absorption such as aluminum monosterate and gelatin.

In some cases, in order to prolong the effect of the active ingredient(s), it is desirable to slow the absorption of the active ingredients from subcutaneous or intramuscular injection. This may be accomplished by the use of a liquid suspension of crystalline or amorphous material having poor water solubility. The rate of absorption of the active ingredients then depends upon its rate of dissolution which, in turn, may depend upon crystal size and crystalline form. Alternatively, delayed absorption of a parenterally-administered active ingredients is accomplished by dissolving or suspending the drug in an oil vehicle.

Injectable depot forms are made by forming microencapsule matrices of the active ingredients in biodegradable polymers such as polylactide-polyglycolide. Depending on the ratio of active ingredients to polymer, and the nature of the particular polymer employed, the rate of release of the active ingredients can be controlled. Examples of other biodegradable polymers include poly(orthoesters) and poly(anhydrides). Depot injectable formulations are also prepared by entrapping the active ingredients in liposomes or microemulsions which are compatible with body tissue. The injectable materials can be sterilized for example, by filtration through a bacterial-retaining filter.

Intracavernosal injection can be carried out by use of a syringe or any other suitable device. An example of a hypodermic syringe useful herein, that can be used for simultaneous injection into both corpora, is described in U.S. Pat. No. 4,127,118. The injection is made on the dorsum of the penis by placement of the needle to the side of each dorsal vein and inserting it deep into the corpora.

The active ingredients can be administered in a pharmaceutical formulation suitable for transurethral drug delivery. The formulation contains one or more selected carriers or excipients, such as water, silicone, waxes, petroleum jelly, polyethylene glycol, propylene glycol, liposomes, sugars such as mannitol and lactose, and/or a variety of other materials, with polyethylene glycol and derivatives thereof particularly preferred. It may be desirable to incorporate a transurethral permeation enhancer in the urethral dosage form. Examples of suitable transurethral permeation enhancers include dimethylsulfoxide, dimethyl formaminde, N,N-dimethylacetamide, decylmethylsulfoxide, polyethylene glycol monolaurate, glycerol monolaurate, lecithin, the 1-substituted azacycloheptan-2-ones, particularly 1-n-dodecylcyclazacycloheptan-2-one (available under the trademark Azone^{®} from Nelson Research & Development Co., Irvine, Calif.), SEPA^{®} (available from Macrochem Co., Lexington, Mass.), alcohols (*e.g*., ethanol), detergents (such as Tergitol^{®}, Nonoxynol-9^{®} and TWEEN-80^{®}) and the like. Transurethral formulations may additionally include one or more enzyme inhibitors effective to inhibit drug-degrading enzymes which may be present in the urethra. Additional optional components include excipients, preservatives (*e.g*., antioxidants), chelating agents, solubilizing agents (*e.g*., surfactants), and the like, as will be appreciated by those skilled in the art of drug formulation preparation and delivery.

Transurethral drug administration, as explained in PCT application WO 91/16021, can be carried out in a number of different ways using a variety of urethral dosage forms. For example, the drug can be introduced into the urethra from a flexible tube, squeeze bottle, pump or aerosol spray. The drug may also be contained in coatings, pellets or suppositories which are absorbed, melted or bioeroded in the urethra. In certain embodiments, the drug is included in a coating on the exterior surface of a penile insert. Drug delivery devices for administering a drug transurethrally are described in U.S. Patent No. 6,037,360 and PCT application WO 91/16021.

Urethral suppository formulations containing polyethylene glycol or a polyethylene glycol derivative can be used as the urethral dosage form, and may be conveniently formulated using conventional techniques, *e.g*., compression molding, heat molding or the like, as will be appreciated by those skilled in the art and as described in the pertinent literature and pharmaceutical texts. See, for example, Remington: The Science and Practice of Pharmacy, 19th Ed. (Easton, PA: Mack Publishing Co., 1995), which discloses typical methods of preparing pharmaceutical compositions in the form of urethral suppositories. It is also preferred that urethral suppositories contain one or more solubilizing agents (*e.g*., a nonionic, anionic, cationic or amphoteric surfactant) effective to increase the solubility of the active ingredients in the polyethylene glycol or other transurethral vehicle.

It may be desirable to deliver the active ingredients in a urethral dosage form which provides for controlled or sustained release of the agents. In such a case, the dosage form typically comprises a biocompatible, biodegradable material, typically a biodegradable polymer. Examples of such polymers include polyester, polyalkylcyanoacrylate, polyorthoester, polyanhydride, albumin, gelatin and starch. As explained, for example, in PCT application WO 96/40054, these and other polymers can be used to provide biodegradable microparticles which enable controlled and sustained drug release, in turn minimizing the required dosing frequency.

The method of intraurethral administration may involve an "active" delivery mechanism such as iontophoresis, electroporation or phonophorcsis. Devices and methods for delivering drugs in this way are well known in the art. Iontophoretically assisted drug delivery is, for example, described in PCT application WO 96/40054. Briefly, the active agents are driven through the urethral wall by means of an electric current passed from an external electrode to a second electrode contained within or affixed to a urethral probe.

The pharmaceutical formulations may be presented in unit-dose or multi-dose sealed containers, for example, ampules and vials, and may be stored in a lyophilized condition requiring only the addition of the sterile liquid carrier, for example water for injection, immediately prior to use. Ex temporaneous injection sections and suspensions may be prepared from sterile powders, granules and tablets of the type described above.

The invention also provides a kit comprising a tramadol material and a phosphodiesterase inhibitor according to claim 8. The kit may comprise one or more containers, each of which contains a tramadol material and a phosphodiesterase inhibitor. In such a case, the tramadol material and the phosphodiesterase inhibitor are preferably contained in the same pharmaceutical composition. Alternatively, the kit may comprise a container holding the tramadol material and a different container holding the phosphodiesterase inhibitor. Suitable containers include rubes, ampules, vials, bottles, foil packets, the wells of a tray and the molded depression of blister packs. The kit may also comprise instructions for administration of the tramadol material and the phosphodiesterase inhibitor to treat a disease or condition for which treatment with the tramadol material is indicated and to reduce the incidence of side effect related to sexual function in males taking the tramadol material. The container(s) will preferably be contained in a package, such as a box. The instructions may be attached to, or printed on, one of the containers, may be printed on a separate sheet of paper inside the package, or may be attached to or printed on the package.

The invention provides another kit comprising a tramadol material, a phosphodiesterase inhibitor and an apioid antagonist according to claim 9. The kit comprises one or more containers, each of which contains a tramadol material a phosphodiesterase inhibitor, and an opioid antagonist Suitable containers are described above. The kit may also comprise instructions for administration of the tramadol material, the phosphodiesterase inhibitor and the opiois antagonist to treat a disease or condition for which treatment with the tramadol material is indicated and to reduce the incidence of a side effect related to sexual function and a side effect not related to sexual function in males taking the tramadol material. The container(s) will preferably be contained in a package, such as a box. The instructions may be attached to, or printed on, one of the containers, may be printed on a separate sheet of paper inside the package, or may be attached to or printed on the package.

It is to be noted that "a" or "an" entity refers to one or more of that entity. For example, "a container" refers to one or more containers.

### EXAMPLES

### Examples 1-2

Tramadol HCl is an FDA-approved centrally acting analgesic with weak opioid and serotonin uptake inhibition activity. A wide range of side effects have been reported as mild or moderate adverse events in placebo controlled clinical studies with tramadol HCl over the past thirty years.

Recently, it has been reported that tramadol HCl can significantly delay ejaculation in men with premature ejaculation. See U.S. Patent No. 6,974,839, Safarinejad ct al., J. Clin. Psychopharmacol., 26(1):27-31 (February 2006) and Salem et al., J. Sex. Med., 5(1):188-193 (January 2008). A placebo controlled, dose-ranging clinical study that enrolled more than 60 male volunteers with severe premature ejaculation who self administered three different doses of oral tramadol HCl 2 to 5 hours before sexual intercourse was performed on behalf of DMI BioSciences, Inc., Aurora, Colorado. The unpublished results of this clinical trial demonstrated a statistically significant delay in ejaculation at each of the three doses. However, the patient reported outcome responses in this study also reported the following previously unreported mild or moderate adverse events (percentages based on total drug exposures for the three doses): erectile dysfunction (8% - 14%), anorgasmia (3% - 7%), and penile hypoesthesia (4% - 5%). Less common unexpected, drug-related mild or moderate adverse events were decreased libido (<1% - 2%) and decreased orgasmic sensation (0 - <1%). These mild or moderate adverse events related to sexual activity were generally dose-dependent and occurred more frequently in men 40 years of age and older.

In order to investigate whether combinations of tramadol HCl with other drugs might reduce these newly reported mild or moderate side effects of tramadol HCl, male volunteers compared adverse events after taking tramadol HCl alone before sexual intercourse to adverse events experienced while taking a combination of tramadol HCl with an oral phosphodiesterase-5 inhibitor (sildenafil citrate, Viagra®) and a combination of tramadol HCl and sildenafil citrate with low oral doses of a known opioid inhibitor (naltrexone HCl) before sexual intercourse.

In particular, three healthy, heterosexual male subjects (mean age 57) signed informed consents and volunteered to self administer oral tramadol HCl 100mg 2 to 5 hours before sexual intercourse and to record any adverse events. At other times, these volunteers self administered oral tramadol HCl 25 to 100mg combined with oral sildenafil citrate 50mg or oral sildenafil citrate 50mg plus oral naltrexone HCl 2.5 to 10mg 2 to 5 hours before sexual intercourse and recorded any adverse effects. These volunteers had not been exposed to any opioid drugs in the preceding 3 months and the volunteers did not take any opioid drugs or alcohol with any drugs administered in this study.

Tramadol Alone. In this study of tramadol side effects, oral tramadol HCl 25mg or 100mg administered alone 2 to 5 hours before sexual intercourse consistently delayed ejaculation, and substantially more mild or moderate adverse events were reported with the 100mg dose. One subject reported numerous adverse events related to sexual function as well as nasal congestion and constipation after each administration of tramadol HCl 100mg alone and subsequently reduced tramadol HCl to 25mg in order to continue participation in this study. Overall, tramadol HCl 100mg taken alone was associated with the following mild or moderate adverse events related to sexual activity (percentages based on total drug exposures for all three volunteers): decreased orgasmic sensation (57%), erectile dysfunction (43%), penile hypoesthesia (43%), decreased libido (43%), anorgasmia (29%). Tramadol HCl 100mg taken alone was also associated in this study with previously reported mild or moderate adverse events not related to sexual activity, such as constipation (71 %), insomnia (71%), nasal congestion (57%), dry mouth (57%), itching (57%), dizziness (14%), restlessness (29%), nausea(14%), and fatigue (14%) (percentages based on total drug exposures for all three volunteers). The incidence of all types of side effects is high in this study as compared to other studies, most likely because of the ages of all three subjects and the inclusion of the one subject who consistently experienced numerous side effects with tramadol HCl above 25mg and who appears to be especially sensitive to the side effects of tramadol. This reflects the unpublished adverse event data collected in clinical study described above of tramadol for premature ejaculation, which showed that an occasional subject would consistently have numerous and repeated side effects at higher tramadol doses. No serious adverse events occurred, the negative effects on sexual function and all other adverse events were temporary.

Tramadol + Sildenafil Citrate. A combination of tramadol HCl 25mg or 100mg and sildenafil citrate 50mg greatly improved sexual function in each episode compared to tramadol HCl alone and resulted in no reports of any of the newly reported mild or moderate adverse events associated with sexual function (*i.e*., no reports of erectile dysfunction, anorgasmia, penile hypoesthesia, decreased libido, or decreased orgasmic sensation); however other previously reported mild or moderate adverse events such as dizziness, insomnia, restlessness, nausea, dry mouth, fatigue, constipation and nasal congestion were occasionally reported. No serious adverse events occurred and all adverse events were temporary.

Tramadol + Sildenafil Citrate + Naltrexone. A triple combination of sildenafil citrate 50mg, tramadol HCl and naltrexone HCl with naltrexone:tramadol ratios of 2.5mg: 100mg, 5.0mag:100 mg and 10mg:100mg greatly improved sexual function and resulted in no reports of any adverse events associated with sexual activity (i.e. no reports of erectile dysfunction, anorgasmia, penile hypoesthesia, decreased libido, or decreased orgasmic sensation). The only mild or moderate adverse events reported with the triple combination of tramadol HCl, sildenafil citrate and naltrexone HCl with a 5.0mug:100 mg naltrexone:tramadol ratio were dry mouth and fatigue. Additionally, triple combinations of tramadol HCl, sildenafil citrate and naltrexone HCl with the naltrexone:tramadol ratio 10mg:100mg resulted in *no reports of any sexual or previously reported mild or moderate adverse events* such as dizziness, insomnia, restlessness, nausea, dry mouth, fatigue, constipation, itching or nasal congestion with the exception of one transient, mild episode of drowsiness that did not interfere with sexual intercourse. No serious adverse events occurred. Volunteers in this study reported that the triple combination of tramadol HCl, sildenafil citrate and naltrexone HCl provided a better sexual experience (*e.g*., "delayed ejaculation", "strong erection") with essentially no mild or moderate side effects compared to tramadol HCl 100mg alone or tramadol HCl 100mg administered with sildenafil citrate 50mg.

Conclusion. The combination of tramadol HCl with a phosphodiesterase-5 inhibitor, such as sildenafil citrate, markedly reduced the incidence of the newly reported side effects related to sexual function such as erectile dysfunction, anorgasmia, penile hypoesthesia, decreased libido, or decreased orgasmic sensation. However, previously reported mild or moderate side effects continued to be reported by the volunteers. The triple combination of tramadol HCl, sildenafil citrate and naltrexone HCl, using a naltrexone:tramadol ratio of 10mg:100mg, significantly reduced the incidence of *all* side effects, previously reported side effects and newly reported sexual side effects, all of which might otherwise interfere with sexual activity. The triple combination of tramadol HCl, sildenafil citrate and naltrexone HCl, allowed tramadol HCl doses as high as 100mg to be readily tolerated during sexual intercourse without interfering side effects.

## Claims

1. A pharmaceutical composition comprising a pharmaceutically-acceptable carrier and active ingredients, wherein the active ingredients consist of a tramadol material and a phosphodiesterase (PDE) inhibitor selected from one or more of a PDE3, PDE4 and a PDE5 inhibitor.

2. A pharmaceutical composition comprising a pharmaceutically-acceptable carrier, a tramadol material, a phosphodiesterase (PDE) inhibitor selected from one or more of a PDE3, PDE4 and a PDE5 inhibitor, and an opioid antagonist.

3. The composition according to claim 2, wherein the opioid antagonist is naltrexone.

4. The composition according to any one of claims 1 to 3, wherein the tramadol material is tramadol or a pharmaceutically-acceptable form thereof, and the PDE inhibitor is a PDE5 inhibitor.

5. The composition according to claim 4, wherein the tramadol material is *(±)cis-*tramadol or a pharmaceutically-acceptable salt thereof, and the PDE5 inhibitor is sildenafil or a pharmaceutically-acceptable form thereof, vardenafil or a pharmaceutically-acceptable form thereof, tadalafil or a pharmaceutically-acceptable form thereof, or a combination of two or more of the foregoing PDE5 inhibitors.

6. The composition according to claim 5, wherein the tramadol material is *(±)cis-*tramadol hydrochloride, and the PDE5 inhibitor is sildenafil citrate, vardenafil hydrochloride, tadalafil or a combination of two or more of the foregoing PDE5 inhibitors.

7. The composition according to claim 6, wherein the PDE5 inhibitor is sildenafil citrate.

8. A kit wherein:
(a) the kit comprises a container holding active ingredients consisting of (i) a tramadol material and (ii) a phosphodiesterase (PDE) inhibitor selected from one or more of a PDE3, PDE4 and PDE5 inhibitor; or
(b) the kit consists of a first container holding a tramadol material and a second container holding a phosphodiesterase (PDE) inhibitor selected from one or more of a PDE3, PDE4 and PDE5 inhibitor.

9. A kit comprising one or more containers, wherein each container holds a tramadol material, a phosphodiesterase (PDE) inhibitor and, an opioid antagonist.

10. The kit according to claim 9, wherein the opioid antagonist is naltrexone.

11. The kit according to any one of claims 8 to 10, wherein the tramadol material is tramadol or a pharmaceutically-acceptable form thereof, and the PDE inhibitor is a PDE5 inhibitor.

12. The kit according to claim 11, wherein the tramadol material is *(*±*)cis*-tromadol or a. pharmaceutically-acceptable salt thereof, and the PDE5 inhibitor is sildenafil or a pharmaceutically-acceptable form thereof, vardenafil or a pharmaceutically-acceptable form thereof, tadalafil or a pharmaceutically-acceptable form thereof, or a combination of two or more of the foregoing PDE5 inhibitors.

13. The kit according to claim 12, wherein the tramadol material is *(*±*)cis*-tramadol hydrochloride, and the PDE5 inhibitor is sildenafil citrate, vardenafil hydrochloride, tadalafil or a combination of two or more of the foregoing PDE-5 inhibitors.

14. The kit according to claim 1:3, wherein the PDE5 inhibitor is sildenafil citrate.

15. 11 pharmaceutical composition comprising a pharmaceutically-acceptable carrier and active ingredients, wherein the active ingredients consist of (i) one or more of a PDE3, PDE4 and PDE5 inhibitor and (ii) a tramadol material, for use in reducing the incidence of a side effect related to sexual function in a human male taking a tramadol material wherein the side effect is caused by the administration of a tramadol material.

16. The pharmaceutical composition for use according to claim 15, wherein the side effect is erectile dysfunction, anorgasmia, penile hypoesthesia, decreased libido, decreased orgasmic sensation or a combination of two or more of the foregoing.

17. The pharmaceutical composition for use according to claim 15, wherein the side effect is erectile dysfunction.

18. A pharmaceutical composition comprising a pharmaceutically-acceptable carrier and active ingredients, wherein the active ingredients consist of (i) one or more of a PDE3, PDE4 and PDE5 inhibitor and (ii) a tramadol material, for use in the treatment of a disease or condition in a human male for which a tramadol material is an effective treatment, wherein the disease or condition is selected from pain, frequent urination, urinary incontinence, cough, bronchitis, common cold or premature ejaculation.

19. A pharmaceutical composition comprising a pharmaceutically-acceptable carrier, one or more of a PDE3, PDE4 and PDE5 inhibitor, a tramadol material, and an opioid antagonist for use in the treatment of a disease or condition in a human male for which a tramadol material is an effective treatment, wherein the disease or condition is selected from pain, frequent urination, urinary incontinence, cough, bronchitis, common cold or premature ejaculation.

20. The pharmaceutical composition for use according to claim 18 or claim 19, wherein the disease or condition is pain.

21. The pharmaceutical composition for use according to claim 18 or claim 19, wherein the disease or condition is premature ejaculation.

22. The pharmaceutical composition for use according to claim 19, wherein the opioid antagonist is naltrexone.

23. The pharmaceutical composition for use according to any one of claims 15 to 22, wherein the tramadol material is tramadol or a pharmaceutically-acceptable form thereof, and the PDE inhibitor is a PDE5 inhibitor.

24. The pharmaceutical composition for use according to claim 23, wherein the tramadol material is *(*±*)cis*-tramadol or a pharmaceutically-acceptable salt thereof, and the PDE5 inhibitor is sildenafil or a pharmaceutically-acceptable form thereof, vardenafil or a pharmaceutically-acceptable form thereof, tadalafil or a pharmaceutically-acceptable form thereof, or a combination of two or more of the foregoing PDE5 inhibitors.

25. The pharmaceutical composition for use according to claim 24, wherein the tramadol material is *(*±*)cis*-trarnadol hydrochloride, and the PDE5 inhibitor is sildenafil citrate, vardenafil hydrochloride, tadalafil or a combination of two or more of the foregoing PDE5 inhibitors.

26. The pharmaceutical composition for use according to claim 25, wherein the PDE5 inhibitor is sildenafil citrate.

27. A pharmaceutical composition comprising a pharmaceutically-acceptable carrier, a phosphodiesterase (PDE) inhibitor selected from one or more of a PDE3, PDE4 and PDE5 inhibitor, an opioid antagonist, and a tramadol material for use in delaying ejaculation in a human male.

28. The pharmaceutical composition for use according to claim 27, wherein the male has premature ejaculation.

29. The pharmaceutical composition for use according to claim 27 or 28, wherein the tramadol material is tramadol or a pharmaceutically-acceptable form thereof.

30. The pharmaceutical composition for use according to claim 29, wherein the tramadol material is *(*±*)cis*-tramadol or a pharmaceutically-acceptable salt thereof.

31. The pharmaceutical composition for use according to claim 30, wherein the tramadol material is *(*±*)cis*-tramadol hydrochloride.

32. The pharmaceutical composition for use according to any one of claims 27 to 31, wherein the opioid antagonist is naltrexone.

33. The pharmaceutical composition for use according to any one of claims 27 to 32, wherein the PDE inhibitor is a PDE5 inhibitor.

34. The pharmaceutical composition for use according to claim 33, wherein the PDE5 inhibitor is sildenafil or a pharmaceutically-acceptable form thereof, vardenafil or a pharmaceutically-acceptable form thereof, tadalafil or a pharmaceutically-acceptable form thereof, or a combination of two or more of the foregoing PDE5 inhibitors.

35. The pharmaceutical composition for use according to claim 34, wherein the PDE5 inhibitor is sildenafil citrate, vardenafil hydrochloride, tadalafil or a combination of two or more of the foregoing PDE5 inhibitors.

36. The pharmaceutical composition for use according to claim 35, wherein the PDE5 inhibitor is sildenafil citrate.

## Patentansprüche

1. Pharmazeutische Zusammensetzung, die einen pharmazeutisch unbedenklichen Trägerstoff und Wirkstoffe umfasst, wobei die Wirkstoffe aus einem Tramadol-Material und einem Phosphodiesterase-Hemmer (PDE-Hemmer), der aus einem oder mehreren eines PDE-3-, eines PDE-4- und eines PDE-5-Hemmers ausgewählt ist, bestehen.

2. Pharmazeutische Zusammensetzung, die einen pharmazeutisch unbedenklichen Trägerstoff, ein Tramadol-Material, einen Phosphodiesterase-Hemmer (PDE-Hemmer), der aus einem oder mehreren eines PDE-3-, eines PDE-4- und eines PDE-5-Hemmers ausgewählt ist, und einen Opioid-Antagonisten umfasst.

3. Zusammensetzung nach Anspruch 2, wobei der Opioid-Antagonist Naltrexon ist.

4. Zusammensetzung nach einem der Ansprüche 1 bis 3, wobei das Tramadol-Material Tramadol oder eine pharmazeutisch unbedenkliche Form davon ist und der PDE-Hemmer ein PDE-5-Hemmer ist.

5. Zusammensetzung nach Anspruch 4, wobei das Tramadol-Material *(*±*)cis*-Tramadol oder ein pharmazeutisch unbedenkliches Salz davon ist und der PDE-5-Hemmer Sildenafil oder eine pharmazeutisch unbedenkliche Form davon, Vardenafil oder eine pharmazeutisch unbedenkliche Form davon, Tadalafil oder eine pharmazeutisch unbedenkliche Form davon oder eine Kombination von zwei oder mehr der vorstehenden PDE-5-Hemmer ist.

6. Zusammensetzung nach Anspruch 5, wobei das Tramadol-Material *(*±*)cis*-Tramadolhydrochlorid ist und der PDE-5-Hemmer Sildenafilcitrat, Vardenafilhydrochlorid, Tadalafil oder eine Kombination von zwei oder mehr der vorstehenden PDE-5-Hemmer ist.

7. Zusammensetzung nach Anspruch 6, wobei der PDE-5-Hemmer Sildenafilcitrat ist.

8. Kit, wobei:
(a) das Kit einen Behälter umfasst, der Wirkstoffe enthält, die aus (i) einem Tramadol-Material und (ii) einem Phosphodiesterase-Hemmer (PDE-Hemmer), der aus einem oder mehreren eines PDE-3-, PDE-4- oder PDE-5-Hemmers ausgewählt ist, bestehen; oder
(b) das Kit aus einem ersten Behälter, der ein Tramadol-Material enthält, und einem zweiten Behälter, der einen Phosphodiesterase-Hemmer (PDE-Hemmer) enthält, der aus einem oder mehreren eines PDE-3-, PDE-4- oder PDE-5-Hemmers ausgewählt ist, besteht.

9. Kit, das einen oder mehrere Behälter umfasst, wobei jeder Behälter ein Tramadol-Material, einen Phosphodiesterase-Hemmer (PDE-Hemmer) und einen Opioid-Antagonisten enthält.

10. Kit nach Anspruch 9, wobei der Opioid-Antagonist Naltrexon ist.

11. Kit nach einem der Ansprüche 8 bis 10, wobei das Tramadol-Material Tramadol oder eine pharmazeutisch unbedenkliche Form davon ist und der PDE-Hemmer ein PDE-5-Hemmer ist.

12. Kit nach Anspruch 11, wobei das Tramadol-Material *(*±*)cis*-Tramadol oder ein pharmazeutisch unbedenkliches Salz davon ist und der PDE-5-Hemmer Sildenafil oder eine pharmazeutisch unbedenkliche Form davon, Vardenafil oder eine pharmazeutisch unbedenkliche Form davon, Tadalafil oder eine pharmazeutisch unbedenkliche Form davon oder eine Kombination von zwei oder mehr der vorstehenden PDE-5-Hemmer ist.

13. Kit nach Anspruch 12, wobei das Tramadol-Material *(*±*)cis*-Tramadolhydrochlorid ist und der PDE-5-Hemmer Sildenafilcitrat, Vardenafilhydrochlorid, Tadalafil oder eine Kombination von zwei oder mehr der vorstehenden PDE-5-Hemmer ist.

14. Kit nach Anspruch 13, wobei der PDE-5-Hemmer Sildenafilcitrat ist.

15. Pharmazeutische Zusammensetzung, die einen pharmazeutisch unbedenklichen Trägerstoff und Wirkstoffe umfasst, wobei die Wirkstoffe aus (i) einem oder mehreren eines PDE-3-, PDE-4- und PDE-5-Hemmers und (ii) einem Tramadol-Material bestehen, zur Verwendung bei der Verringerung des Auftretens einer Nebenwirkung, die mit der Sexualfunktion bei einem Mann, der ein Tramadol-Material einnimmt, in Zusammenhang steht, wobei die Nebenwirkung von der Verabreichung eines Tramadol-Materials verursacht wird.

16. Pharmazeutische Zusammensetzung zur Verwendung nach Anspruch 15, wobei die Nebenwirkung erektile Dysfunktion, Anorgasmie, penile Hypästhesie, verminderte Libido, vermindertes Orgasmusempfinden oder eine Kombination von zwei oder mehr der vorstehenden ist.

17. Pharmazeutische Zusammensetzung zur Verwendung nach Anspruch 15, wobei die Nebenwirkung erektile Dysfunktion ist.

18. Pharmazeutische Zusammensetzung, die einen pharmazeutisch unbedenklichen Trägerstoff und Wirkstoffe umfasst, wobei die Wirkstoffe aus (i) einem oder mehreren eines PDE-3-, PDE-4- und PDE-5-Hemmers und (ii) einem Tramadol-Material bestehen, zur Verwendung bei der Behandlung einer Erkrankung oder Krankheit bei einem Mann, für die ein Tramadol-Material eine wirksame Behandlung ist, wobei die Erkrankung oder Krankheit aus Schmerzen, häufigem Wasserlassen, Harninkontinenz, Husten, Bronchitis, Erkältung oder vorzeitigem Samenerguss ausgewählt ist.

19. Pharmazeutische Zusammensetzung, die einen pharmazeutisch unbedenklichen Trägerstoff, einen oder mehrere eines PDE-3-, PDE-4- und PDE-5-Hemmers, ein Tramadol-Material und einen Opioid-Antagonisten umfasst, zur Verwendung bei der Behandlung einer Erkrankung oder Krankheit bei einem Mann, für die ein Tramadol-Material eine wirksame Behandlung ist, wobei die Erkrankung oder Krankheit aus Schmerzen, häufigem Wasserlassen, Harninkontinenz, Husten, Bronchitis, Erkältung oder vorzeitigem Samenerguss ausgewählt ist.

20. Pharmazeutische Zusammensetzung zur Verwendung nach Anspruch 18 oder 19, wobei die Erkrankung oder Krankheit Schmerzen ist.

21. Pharmazeutische Zusammensetzung zur Verwendung nach Anspruch 18 oder 19, wobei die Erkrankung oder Krankheit vorzeitiger Samenerguss ist.

22. Pharmazeutische Zusammensetzung zur Verwendung nach Anspruch 19, wobei der Opioid-Antagonist Naltrexon ist.

23. Pharmazeutische Zusammensetzung zur Verwendung nach einem der Ansprüche 15 bis 22, wobei das Tramadol-Material Tramadol oder eine pharmazeutisch unbedenkliche Form davon ist und der PDE-Hemmer ein PDE-5-Hemmer ist.

24. Pharmazeutische Zusammensetzung zur Verwendung nach Anspruch 23, wobei das Tramadol-Material *(*±*)cis-*Tramadol oder ein pharmazeutisch unbedenkliches Salz davon ist und der PDE-5-Hemmer Sildenafil oder eine pharmazeutisch unbedenkliche Form davon, Vardenafil oder eine pharmazeutisch unbedenkliche Form davon, Tadalafil oder eine pharmazeutisch unbedenkliche Form davon oder eine Kombination von zwei oder mehr der vorstehenden PDE-5-Hemmer ist.

25. Pharmazeutische Zusammensetzung zur Verwendung nach Anspruch 24, wobei das Tramadol-Material *(*±*)cis-*Tramadolhydrochlorid ist und der PDE-5-Hemmer Sildenafilcitrat, Vardenafilhydrochlorid, Tadalafil oder eine Kombination von zwei oder mehr der vorstehenden PDE-5-Hemmer ist.

26. Pharmazeutische Zusammensetzung zur Verwendung nach Anspruch 25, wobei der PDE-5-Hemmer Sildenafilcitrat ist.

27. Pharmazeutische Zusammensetzung, die einen pharmazeutisch unbedenklichen Trägerstoff, einen Phosphodiesterase-Hemmer (PDE-Hemmer), der aus einem oder mehreren eines PDE-3-, PDE-4- und PDE-5-Hemmers s ausgewählt ist, einen Opioid-Antagonisten und ein Tramadol-Material umfasst, zur Verwendung bei der Verzögerung des Samenergusses bei einem Mann.

28. Pharmazeutische Zusammensetzung zur Verwendung nach Anspruch 27, wobei der Mann unter vorzeitigem Samenerguss leidet.

29. Pharmazeutische Zusammensetzung zur Verwendung nach Anspruch 27 oder 28, wobei das Tramadol-Material Tramadol oder eine pharmazeutisch unbedenkliche Form davon ist.

30. Pharmazeutische Zusammensetzung zur Verwendung nach Anspruch 29, wobei das Tramadol-Material *(*±*)cis-*Tramadol oder ein pharmazeutisch unbedenkliches Salz davon ist.

31. Pharmazeutische Zusammensetzung zur Verwendung nach Anspruch 30, wobei das Tramadol-Material *(*±*)cis-*Tramadolhydrochlorid ist.

32. Pharmazeutische Zusammensetzung zur Verwendung nach einem der Ansprüche 27 bis 31, wobei der Opioid-Antagonist Naltrexon ist.

33. Pharmazeutische Zusammensetzung zur Verwendung nach einem der Ansprüche 27 bis 32, wobei der PDE-Hemmer ein PDE-5-Hemmer ist.

34. Pharmazeutische Zusammensetzung zur Verwendung nach Anspruch 33, wobei der PDE-5-Hemmer Sildenafil oder eine pharmazeutisch unbedenkliche Form davon, Vardenafil oder eine pharmazeutisch unbedenkliche Form davon, Tadalafil oder eine pharmazeutisch unbedenkliche Form davon oder eine Kombination von zwei oder mehr der vorstehenden PDE-5-Hemmer ist.

35. Pharmazeutische Zusammensetzung zur Verwendung nach Anspruch 34, wobei der PDE-5-Hemmer Sildenafilcitrat, Vardenafilhydrochlorid, Tadalafil oder eine Kombination von zwei oder mehr der vorstehenden PDE-5-Hemmer ist.

36. Pharmazeutische Zusammensetzung zur Verwendung nach Anspruch 35, wobei der PDE-5-Hemmer Sildenafilcitrat ist.

## Revendications

1. Composition pharmaceutique comprenant un véhicule pharmaceutiquement acceptable et des principes actifs, dans laquelle les principes actifs sont constitués d'une substance à base de tramadol et d'un inhibiteur de phosphodiestérase (PDE) représentant un ou plusieurs éléments choisis parmi le groupe comprenant un inhibiteur de PDE3, de PDE4 et de PDE5.

2. Composition pharmaceutique comprenant un véhicule pharmaceutiquement acceptable, une substance à base de tramadol, un inhibiteur de phosphodiestérase (PDE) représentant un ou plusieurs éléments choisis parmi le groupe comprenant un inhibiteur de PDE3, de PDE4 et de PDE5, et un antagoniste opioïde.

3. Composition selon la revendication 2, dans laquelle l'antagoniste opioïde est la naltrexone.

4. Composition selon l'une quelconque des revendications 1 à 3, dans laquelle la substance à base de tramadol est le tramadol ou une forme pharmaceutiquement acceptable de celui-ci, et l'inhibiteur de PDE est un inhibiteur de PDE5.

5. Composition selon la revendication 4, dans laquelle la substance à base de tramadol est le *(*±*)-cis*-tramadol ou un sel pharmaceutiquement acceptable de celui-ci, et l'inhibiteur de PDE5 est le sildénafil ou une forme pharmaceutiquement acceptable de celui-ci, le vardénafil ou une forme pharmaceutiquement acceptable de celui-ci, le tadalafil ou une forme pharmaceutiquement acceptable de celui-ci, ou une combinaison de deux ou plusieurs des inhibiteurs de PDE5 ci-dessus.

6. Composition selon la revendication 5, dans laquelle la substance à base de tramadol est le chlorhydrate de *(*±*)-cis*-tramadol, et l'inhibiteur de PDE5 est le citrate de sildénafil, le chlorhydrate de vardénafil, le tadalafil ou une combinaison de deux ou plusieurs des inhibiteurs de PDE5 ci-dessus.

7. Composition selon la revendication 6, dans laquelle l'inhibiteur de PDE5 est le citrate de sildénafil.

8. Trousse dans laquelle :
(a) la trousse comprend un contenant renfermant des principes actifs constitués de (i) une substance à base de tramadol et (ii) un inhibiteur de phosphodiestérase (PDE) représentant un ou plusieurs éléments choisis parmi le groupe comprenant un inhibiteur de PDE3, de PDE4 et de PDE5 ; ou
(b) la trousse comprend un premier contenant renfermant une substance à base de tramadol et un second contenant renfermant un inhibiteur de phosphodiestérase (PDE) représentant un ou plusieurs éléments choisis parmi le groupe comprenant un inhibiteur de PDE3, de PDE4 et de PDE5.

9. Trousse comprenant un ou plusieurs contenants, dans laquelle chaque contenant renferme une substance à base de tramadol, un inhibiteur de phosphodiestérase (PDE) et un antagoniste opioïde.

10. Trousse selon la revendication 9, dans laquelle l'antagoniste opioïde est la naltrexone.

11. Trousse selon l'une quelconque des revendications 8 à 10, dans laquelle la substance à base de tramadol est le tramadol ou une forme pharmaceutiquement acceptable de celui-ci, et l'inhibiteur de PDE est un inhibiteur de PDE5.

12. Trousse selon la revendication 11, dans laquelle la substance à base de tramadol est le *(*±*)-cis*-tramadol ou un sel pharmaceutiquement acceptable de celui-ci, et l'inhibiteur de PDE5 est le sildénafil ou une forme pharmaceutiquement acceptable de celui-ci, le vardénafil ou une forme pharmaceutiquement acceptable de celui-ci, le tadalafil ou une forme pharmaceutiquement acceptable de celui-ci, ou une combinaison de deux ou plusieurs des inhibiteurs de PDE5 ci-dessus.

13. Trousse selon la revendication 12, dans laquelle la substance à base de tramadol est le chlorhydrate de *(*±*)-cis*-tramadol, et l'inhibiteur de PDE5 est le citrate de sildénafil, le chlorhydrate de vardénafil, le tadalafil ou une combinaison de deux ou plusieurs des inhibiteurs de PDE5 ci-dessus.

14. Trousse selon la revendication 13, dans laquelle l'inhibiteur de PDE5 est le citrate de sildénafil.

15. Composition pharmaceutique comprenant un véhicule pharmaceutiquement acceptable et des principes actifs, dans laquelle les principes actifs sont constitués de (i) un ou plusieurs éléments choisis parmi le groupe comprenant un inhibiteur de PDE3, de PDE4 et de PDE5 et (ii) une substance à base de tramadol, destinée à être utilisée pour réduire l'incidence d'un effet secondaire lié à la fonction sexuelle chez un homme prenant une substance à base de tramadol, l'effet secondaire étant causé par l'administration d'une substance à base de tramadol.

16. Composition pharmaceutique destinée à être utilisée selon la revendication 15, dans laquelle l'effet secondaire est un dysfonctionnement érectile, une anorgasmie, une hypoesthésie pénienne, une diminution de la libido, une diminution de la sensation de l'orgasme ou une combinaison de deux ou plusieurs des effets secondaires précédents.

17. Composition pharmaceutique destinée à être utilisée selon la revendication 15, dans laquelle l'effet secondaire est un dysfonctionnement érectile.

18. Composition pharmaceutique comprenant un véhicule pharmaceutiquement acceptable et des principes actifs, dans laquelle les principes actifs sont constitués de (i) un ou plusieurs éléments choisis parmi le groupe comprenant un inhibiteur de PDE3, de PDE4 et de PDE5 et (ii) une substance à base de tramadol, destinée à être utilisée pour le traitement d'une maladie ou d'un état pathologique chez un homme pour qui une substance à base de tramadol est un traitement efficace, la maladie ou l'état pathologique étant choisi parmi la douleur, une miction fréquente, une incontinence urinaire, une toux, une bronchite, un rhume banal ou une éjaculation précoce.

19. Composition pharmaceutique comprenant un véhicule pharmaceutiquement acceptable, un ou plusieurs éléments choisis parmi le groupe comprenant un inhibiteur de PDE3, de PDE4 et de PDE5, une substance à base de tramadol, et un antagoniste opioïde, destinée à être utilisée pour le traitement d'une maladie ou d'un état pathologique chez un homme pour qui une substance à base de tramadol est un traitement efficace, la maladie ou l'état pathologique étant choisi parmi la douleur, une miction fréquente, une incontinence urinaire, une toux, une bronchite, un rhume banal ou une éjaculation précoce.

20. Composition pharmaceutique destinée à être utilisée selon la revendication 18 ou la revendication 19, dans laquelle la maladie ou l'état pathologique est la douleur.

21. Composition pharmaceutique destinée à être utilisée selon la revendication 18 ou la revendication 19, dans laquelle la maladie ou l'état pathologique est une éjaculation précoce.

22. Composition pharmaceutique destinée à être utilisée selon la revendication 19, dans laquelle l'antagoniste opioïde est la naltrexone.

23. Composition pharmaceutique destinée à être utilisée selon l'une quelconque des revendications 15 à 22, dans laquelle la substance à base de tramadol est le tramadol ou une forme pharmaceutiquement acceptable de celui-ci, et l'inhibiteur de PDE est un inhibiteur de PDE5.

24. Composition pharmaceutique destinée à être utilisée selon la revendication 23, dans laquelle la substance à base de tramadol est le *(*±*)-cis*-tramadol ou un sel pharmaceutiquement acceptable de celui-ci, et l'inhibiteur de PDE5 est le sildénafil ou une forme pharmaceutiquement acceptable de celui-ci, le vardénafil ou une forme pharmaceutiquement acceptable de celui-ci, le tadalafil ou une forme pharmaceutiquement acceptable de celui-ci, ou une combinaison de deux ou plusieurs des inhibiteurs de PDE5 ci-dessus.

25. Composition pharmaceutique destinée à être utilisée selon la revendication 24, dans laquelle la substance à base de tramadol est le chlorhydrate de *(*±*)-cis*-tramadol, et l'inhibiteur de PDE5 est le citrate de sildénafil, le chlorhydrate de vardénafil, le tadalafil ou une combinaison de deux ou plusieurs des inhibiteurs de PDE5 ci-dessus.

26. Composition pharmaceutique destinée à être utilisée selon la revendication 25, dans laquelle l'inhibiteur de PDE5 est le citrate de sildénafil.

27. Composition pharmaceutique comprenant un véhicule pharmaceutiquement acceptable, un inhibiteur de phosphodiestérase (PDE) représentant un ou plusieurs éléments choisis parmi le groupe comprenant un inhibiteur de PDE3, de PDE4 et de PDE5, un antagoniste opioïde, et une substance à base de tramadol, destinée à être utilisée pour retarder l'éjaculation chez un homme.

28. Composition pharmaceutique destinée à être utilisée selon la revendication 27, dans laquelle l'homme a une éjaculation précoce.

29. Composition pharmaceutique destinée à être utilisée selon la revendication 27 ou 28, dans laquelle la substance à base de tramadol est le tramadol ou une forme pharmaceutiquement acceptable de celui-ci.

30. Composition pharmaceutique destinée à être utilisée selon la revendication 29, dans laquelle la substance à base de tramadol est le *(*±*)-cis*-tramadol ou un sel pharmaceutiquement acceptable de celui-ci.

31. Composition pharmaceutique destinée à être utilisée selon la revendication 30, dans laquelle la substance à base de tramadol est le chlorhydrate de *(*±*)-cis*-tramadol.

32. Composition pharmaceutique destinée à être utilisée selon l'une quelconque des revendications 27 à 31, dans laquelle l'antagoniste opioïde est la naltrexone.

33. Composition pharmaceutique destinée à être utilisée selon l'une quelconque des revendications 27 à 32, dans laquelle l'inhibiteur de PDE est un inhibiteur de PDE5.

34. Composition pharmaceutique destinée à être utilisée selon la revendication 33, dans laquelle l'inhibiteur de PDE5 est le sildénafil ou une forme pharmaceutiquement acceptable de celui-ci, le vardénafil ou une forme pharmaceutiquement acceptable de celui-ci, le tadalafil ou une forme pharmaceutiquement acceptable de celui-ci, ou une combinaison de deux ou plusieurs des inhibiteurs de PDE5 ci-dessus.

35. Composition pharmaceutique destinée à être utilisée selon la revendication 34, dans laquelle l'inhibiteur de PDE5 est le citrate de sildénafil, le chlorhydrate de vardénafil, le tadalafil ou une combinaison de deux ou plusieurs des inhibiteurs de PDE5 ci-dessus.

36. Composition pharmaceutique destinée à être utilisée selon la revendication 35, dans laquelle l'inhibiteur de PDE5 est le citrate de sildénafil.
